# EUROPÄISCHE PATENTSCHRIFT

(11) **EP 0 630 248 B1**
(45) Veröffentlichungstag und Bekanntmachung des Hinweises auf die Patenterteilung: **14.10.1998**
(21) Anmeldenummer: 93920566.2
(22) Anmeldetag: 18.03.1993
(51) Int. Cl.: A61K 31/565, A61K 9/70

(54) **VERWENDUNG VON ÖSTRIOL ZUR HERSTELLUNG EINEN TRANSDERMALEN THERAPEUTISCHEN SYSTEME ZUR BEHANDLUNG VON KLIMAKTERISCHER OSTEOPOROSE**
USE OF ESTRIOL FOR THE MANUFACTURE OF A TRANSDERMAL THERAPEUTIC SYSTEM FOR TREATING CLIMACTERIC OSTEOPOROSIS
UTILISATION D' OESTRIOL POUR LA MANUFACTURE D'UN SYSTEME THERAPEUTIQUE TRANSDERMAL POUR TRAITER L'OSTEOPOROSE CLIMACTERIQUE

(30) Priorität: 21.03.1992 DE 4209295
(43) Veröffentlichungstag der Anmeldung: 28.12.1994
(73) Patentinhaber: EnTec Gesellschaft für endokrinologische Technologie m.b.H., D-22529 Hamburg (DE)
(72) Erfinder: ELGER, Walter, D-1000 Berlin 33 (DE); SCHNEIDER, Birgitt, D-6900 Jena (DE); OETTEL, Michael, D-6900 Jena (DE); ERNST, Michael, D-6902 Jena/Lobeda Ost (DE); HÜBLER, Doris, / (DE); DITTGEN, Michael, D-5020 Apolda (DE)
(74) Vertreter: UEXKÜLL & STOLBERG
(86) Internationale Anmeldenummer: EP9300636
(87) Internationale Veröffentlichungsnummer: WO9318774

(56) Entgegenhaltungen:
- EP-A- 0 370 220
- DE-A- 3 910 578
- Week 9252, Derwent Publications Ltd., London, GB; AN 92-427154
- DRUGS Bd. 39, Nr. 2, 1990, Seiten 203 - 217 SITRUK-WARE, R., 'Estrogen therapy during menopause'
- MATURITAS Bd. 12, Nr. 3, 1990, Seiten 199 - 214 LAURITZEN, C., 'Clinical use of oestrogens and progestogens.'

## Beschreibung

Die Erfindung betrifft die Verwendung von Östriol als alleinigem Wirkstoff zur Herstellung eines transdermalen, den Wirkstoff kontinuierlich freisetzenden Arzneimittels zur Behandlung von klimakterischer Osteoporose.

Der Ausfall der weiblichen Geschlechtshormone - Östrogene - im Klimakterium kann zu Erscheinungen führen, die therapiebedürftig sind. Östrogene sind Steroidhormone, die sich von dem tetracyklischen C₁₈-Steroid Östran ableiten. Unter den natürlichen Östrogenen unterscheidet man Östron (E₁), Östradiol (E₂) und Östriol (E₃), wobei Östron und Östriol als die physiologisch wichtigsten gelten. Eine Hormonsubstitution mit diesen beiden Östrogenen führt schnell zu einer Verbesserung der psychischen Befindlichkeit und langfristig zu einer günstigen Beeinflussung des Knochen- und Lipidstoffwechsels. Die letztgenannten Faktoren stellen eine wirksame Prävention von Erkrankungen des Skelettsystems und von Erkrankungen des Herzkreislaufsystems dar. Es ist ohne jeden Zweifel erwiesen, daß E₁ und E₂ die im Klimakterium fortschreitende Osteoporose verhindern und das Fortschreiten arteriosklerotischer Gefäßveränderungen verlangsamen können.

Den genannten günstigen Effekten steht ein für tolerierbar gehaltenes Risiko durch mögliche stimulierende Effekte der Östrogene auf das Wachstum hormonabhängiger Tumore des Genitaltraktes (Endometrium) und der Brustdrüse gegenüber. Die bekannte Kombination von Östrogenen mit Gestagenen verfolgt das Ziel, entsprechende Risiken durch die anti-proliferative Wirkung von Gestagenen im Uterus zu minimieren.

Beipiele für Therapien mit natürlichen Östrogenen sind:
1) Die transdermale Applikation von Östradiol (beispielsweise Estraderm® TTS),
2) die orale Applikation von Östradiol und seinen Estern,
3) die orale Applikation von konjugierten Östrogenen; diese werden aus dem Harn gravider Stuten gewonnen und sie stellen eine Mischung verschiedener Östrogene dar und
4) die vaginale Applikation natürlicher Östrogene. Letztere verfolgt das Ziel, die Atrophie der Genitalschleimhäute zu beheben.

Wie oben erläutert, ist nachteilig an der Therapie mit derartigen Östrogenen deren Eigenschaft, Uteruskrebs (Endometriumskarzinom) oder Brustkrebs zu erzeugen.

Es ist erwiesen, daß durch Kombination der oben genannten Östrogene mit einem Gestagen das Risiko der Betroffenen, an einem Endometriumskarzinom zu erkranken, auf 1/6 des Risikos von nur mit Östrogenen behandelten Frauen gesenkt wird. Beispiele für derartige Kombinationstherapien sind in DE-A-39 10 578, DE-A-39 08 130, DE 38 36 826 und EP 0 474 374 beschrieben.

Von einer entsprechenden Östrogen-Gestagen Kombination ist jedoch ein günstiger Effekt auf die Mortalität durch Mammakarzinome nicht zu erwarten. (L.A. Brinton "Menopause and ..." New York, Academy of Sciences, **592**, 357-362, (1990); R.A. Lobo, "Estrogen and Cardiovascular Disease" in "Multidisciplinary Perspectives on Menopause", herausgegeben von M. Flint, F. Kronenberg und W. Utian, Annals of The N.Y. Acad. of Sciences, **592**, Seiten 286-294 (1990)).

Brustkrebs ist bei Frauen die häufigste Krebserscheinung überhaupt. Generell ist die These akzeptiert, daß der mitogene Effekt der Summe der im Verlauf des Lebens auf die Brustdrüse einwirkenden Östrogenmenge ein entscheidener (Risiko-)Faktor für das Auftreten einer Krebserkrankung dieses Organs ist (vgl. B.E. Henderson, R. Ross und L. Bernstein "Estrogens as a cause of human cancer". The Richard and Hinda Rosenthal Foundation Award Lecture. Cancer Res., **48**, 246-253, (1988); R. Clarke, R.B. Dickson und M.E. Lippman "The role of steroid hormones and growth factors in the control of normal and malignant breast" in "Nuclear Hormone Receptors", herausgegeben von M.G. Parker, Academic Press, London, Seiten 297-319 (1991)).

Die Gefahr der Risiko-Steigerung bezüglich einer Erkrankung an Brustkrebs ist daher ein zentraler Faktor bei der Abschätzung von Nutzen und Risiko der Hormon-Substitutions-Therapie (HRT-Hormon Replacement Therapy; L.A. Brinton "Menopause and the risk of breast cancer", in Multidisciplinary perspectives on Menopause. Ann, N.Y. Academy of Sciences, herausgegeben von M. Flint, F. Kronenberg und W. Utian, **592**, Seiten 357-362 (1990) review). Bei länger dauernder Therapie lieferten verschiedene epidemiologische Studien Anhaltspunkte für eine Erhöhung des relativen Risikos um den Faktor 1,5 bis 3,1, letzteres bei über 10jähriger Dauer der HRT (Tabelle 3, Seite 359 der oben genannten Arbeit).

Anders ist die Situation bei Östriol. Von diesem ist einerseits bekannt, daß es die Entwicklung von Mammakarzinomen verhindern bzw. unterdrücken kann, andererseits wurde ihm bisher eine unzureichende therapeutische Wirksamkeit im Rahmen der Substitutionstherapie (HRT) zugesprochen. Letzteres gilt in besonderem Maße für die Prävention der Osteoporose. So wird in einer offiziellen Stellungnahme der Deutschen Gesellschaft für Endokrinologie die Unwirksamkeit von Östriol zu Osteoporose-Prophylaxe ausdrücklich hervorgehoben (vgl. Deutsches Ärzteblatt - Ärztliche Mitteilungen, 85, 1322-1325, insb. 1325, li. SP. (1988)).

Östriol wird normalerweise im Zyklus und in der Schwangerschaft gebildet, wobei während der Gravidität außerordentlich große Mengen ausgeschüttet werden. Pharmakologisch gilt Östriol als schwach wirksames Östrogen. Die Unterschiede im Vergleich zu E₁ und E₂ sowie zu anderen synthetischen Östrogenen zeigen sich beispielsweise darin, daß Östriol bei einmaliger Applikation bei ovarektomierten Ratten die Uterusgewichte nicht signifikant stimuliert. Entsprechende Befunde, die für fehlende oder geringe Wirkungen auf den Uterus sprechen, sind auch in Untersuchungen bei Frauen erhoben worden. Selbst vergleichsweise hohe Dosierungen von 5 mg E₃/Tag oral (H.W. Rudel und R.A. Kincl "Oral contractives. Human fertility studies and side effects" in "International Encyclopedia of Pharmacology and Therapeutics", Kapitel 34, "Pharmacology of the Endocrine System and related drugs...", herausgegeben von M. Tausk, Band II, Seiten 385 ff., Pergamon Press, London, (1972)) waren nicht in der Lage, die Ovulation bei Frauen zu hemmen, während dies mit Östradiol und konjugierten Östrogenen selbst bei niedrigeren Dosierungen eindeutig möglich war.

In anderen Versuchsanordnungen, in denen Östriol in Kombination mit "starken" Östrogenen appliziert wurde, erwies diese Substanz sich jedoch als antiöstrogen wirksam. Östriol ist demgemäß selbst östrogen, es wirkt jedoch über die Verdrängung stärkerer Östrogene von dem Rezeptor antiöstrogen.

Insbesondere läßt die erwiesene Antitumorwirkung von Östriol in der Induktions- und in der Promotionsphase der Tumorentwicklung (H.M. Lemon "Antimammary cancerogenic Activity of 17α-Ethinyl-Estriol", Cancer **60**, 2873-2881 (1987)) erwarten, daß von Therapeutika, die Östriol in pharmakodynamisch relevanter Formulierung enthalten, kein zusätzlicher Wachstumsimpuls auf Tumorherde ausgeht, sondern, daß im Gegenteil eine entsprechende Therapie zu einer Reduktion des Mammatumorrisikos führt.

Die gegenüber anderen Östrogenen möglicherweise nicht nur unproblematischen sondern sogar antikanzerogenen Eigenschaften von Östriol wurden u.a. auch von Follingstad in "The Journal of the American Medical Association" 239, 29/30 (1978) diskutiert und es wurde die klinische Erprobung dieses Östrogens empfohlen. Einschlägige umfangreiche Untersuchungen über einen Zeitraum von 2 Jahren wurden von Lindsay et al. (R. Lindsay, D.M. Hart, A. MacLean, J. Garwood, A.C. Clark und A. Kraszewski (1979) Boneloss during oestriol therapy in postmenopausal women. Maturitas 1: 179) mit **80** Frauen im postmenopausalen Stadium durchgeführt und die Ergebnisse wurden kontinuierlich über den Versuchszeitraum insbesondere im Hinblick auf eine mögliche antiosteoporotische Wirksamkeit der Substanz anhand der KnochenMineral-Gehaltes ausgewertet. Zur Enttäuschung der Autoren zeigte es sich, daß Östriol auch in Dosismengen von 12 mg/Tag oral keine osteoprotektive Wirksamkeit am Knochen der behandelten Frauen entfaltete. Die Unwirksamkeit von Östriol am Knochen ist inzwischen als Standardwissen in die einschlägigen Lehrbücher eingegangen (vgl. beispielsweise Freimut A. Leidenberger, "Klinische Endokrinologie für Frauenärzte" Springer Verlag 1992, wo es auf Seite 356 wörtlich heißt:

### Nicht wirksam zur Osteoporoseprophylaxe:

- Östriol, das Medikament kann jedoch psychovegetative Symptome und lokale Befunde im Genitalbereich beheben.

Auf die Unwirksamkeit von Östriol für die hier zur Diskussion stehende Indikation wird ferner auch in Produktinformationen für Präparate hingewiesen, die Östriol als Wirkstoff enthalten (vgl. beispielsweise Jenapharm Arzneimittel: Sortima und Preise vom 01.07.1991, Seite 67).

Diese Gesamtbeurteilung wurde auch durch den Bericht von Blum aus dem Jahre 1985 nicht beeinflußt (vgl. M. Blum, Clin. Exp. Obstet. Gynecol. 12, 1/2 (1985)), welcher eine Studie an 25 Frauen im postmenopausalen Stadium beschreibt. Den Frauen wurde eine 0,5 mg Östriol/g enthaltende Vaginalcreme 2 Wochen lang täglich und nachfolgend zweimal wöchentlich in einer Menge von jeweils 0,5g Creme über einen Zeitraum von 4 Monaten vor dem Schlafengehen verabreicht. Es wurde eine Besserung eines Teils der postmenopausalen Störungen, insbesondere der Störungen im Zusammenhang mit genitourinärer Atrophie und Dysparurenie beobachtet während Schlaflosigkeit, Schweißausbrüche und aufsteigende Hitze sich nicht bessern ließen. Es war demgemäß erforderlich, zur Bekämpfung dieser Symptome zusätzlich Chlonidin zu verabreichen. Der Autor untersuchte ferner das Verhältnis von Calcium zu Kreatenin und schloß aus einer geringfügigen Senkung dieses Verhältnisses während des Untersuchungszeitraums auf eine anti-osteoporotische Wirkung des Östriols. Für eine derartige Aussage war jedoch der Untersuchungszeitraum viel zu kurz, vgl. Lindsay et al. a.a. O. und im übrigen ist dieses Verhältnis allein nicht aussagekräftig, da es unter anderem ernährungsabhängig ist; Untersuchungen am Knochen sind von Blum nicht durchgeführt bzw. nicht berichtet worden.

Die Tatsache, daß alle kardialen Symptome der postmenopausalen Störungen in der von Blum berichteten Studie von Östriol unbeeinflußt blieben mit dem Ergebnis, daß zusätzlich Chlonidin verabreicht werden mußte, läßt darauf schließen, daß die Patientinnen nach wie vor unter einem Östrogendefizit litten.

Bei Würdigung des gesamten Standes der Technik läßt sich somit feststellen, daß bisher Östriol in der Fachwelt als ungeeignet zur Behandlung klimakterischer Osteoporose gilt.

Aufgabe der Erfindung ist es, Mittel zu schaffen, mit deren Hilfe die klimakterische Osteoporose sich wirksam bekämpfen läßt ohne gleichzeitig eine Steigerung des Risikos der Erkrankung an Brustkrebs in Kauf nehmen zu müssen.

Zur Lösung der Aufgabe wird erfindungsgemäß die Verwendung von Östriol zur Herstellung transdermalen Retard-Präparaten vorgeschlagen, die eine kontinuierliche Wirkstofffreisetzung über mindestens 24 Stunden gewährleisten.

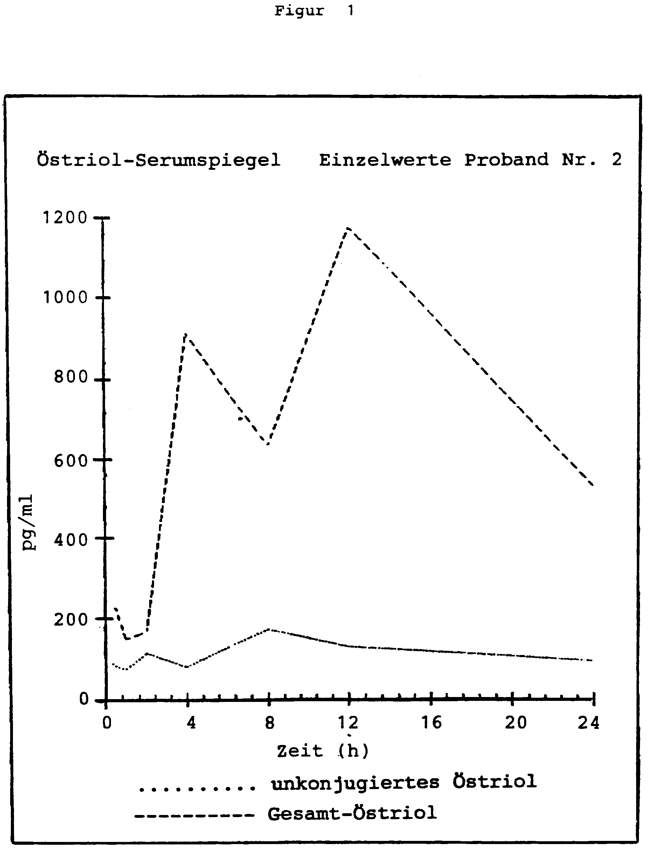

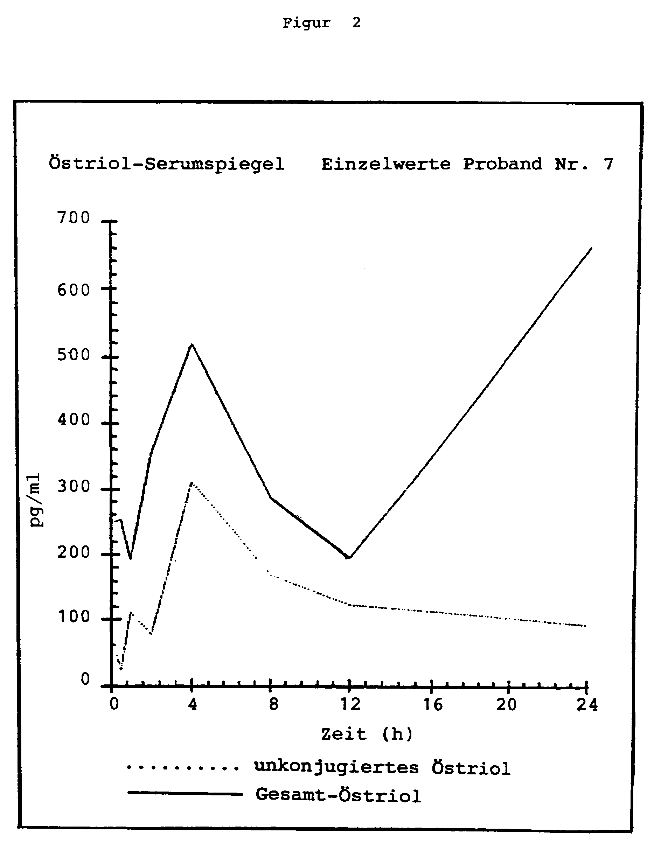

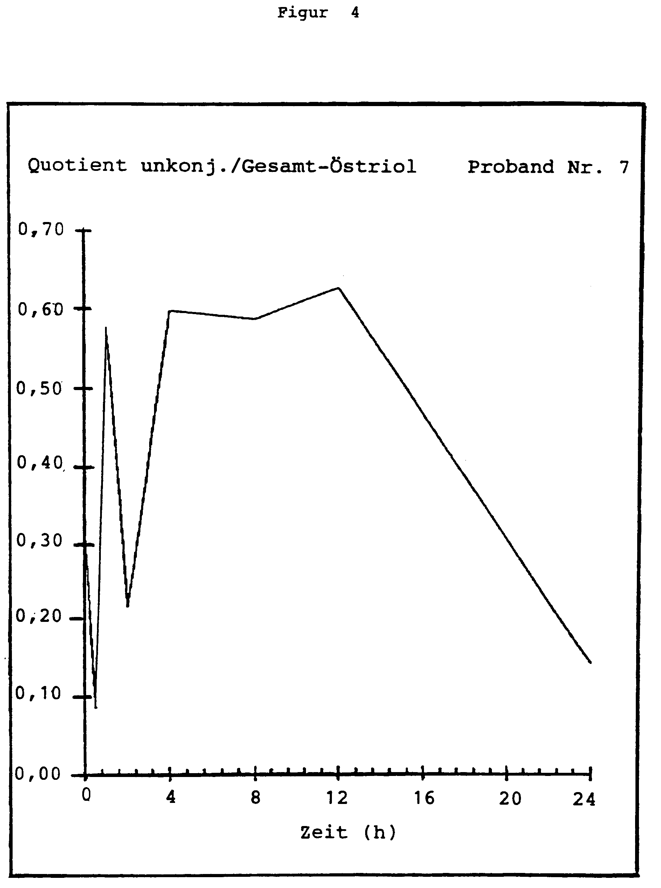

Erfindungsgemäß hat es sich überraschenderweise gezeigt, daß Östriol ein starkes und anti-osteoporotisch wirksames Östrogen ist, wenn es transdermal in einem System verabreicht wird, welches den Wirkstoff mindestens 24 Stunden lang kontinuierlich abgibt. Basis der Erfindung ist der überraschende Befund, daß bei kontinuierlicher Verabreichung von E₃ ein unerwartetes Phänomen hinsichtlich des Blutspiegels für den aktiven Wirkstoff eintritt. Östrogene und somit auch Östriol werden in der Leber durch Konjugation mit Glucuronsäure oder Sulfat inaktiviert. Alle bisherigen Befunde deuten darauf hin, daß Östriol extrem schnell inaktiviert wird (vgl. H. Kuhl, "Pharmacokinetics of oestrogens and progesterons", MATURITAS 12, 171-197, insbes. Fig.4, S.178 (1990)). Erfindungsgemäß hat es sich jedoch gezeigt, daß sich bei kontinuierlicher Verabreichung des Wirkstoffes ein weitgehend konstanter Spiegel der aktiven Form der Substanz einstellt. Dieser ist offenbar unabhängig von der Gesamtmenge aus freiem und konjugiertem Wirkstoff (siehe unten) und scheint einem, durch die kontinuierliche Verabreichung aktiviertem endogenen Regelmechanismus zu gehorchen. So wurde bei transdermaler Verabreichung im humanen Stichversuch (vgl. Beispiel 6) die überraschende Beobachtung gemacht, daß nach einem initialen Anstieg die Konzentration des nicht-metabolisierten Hormons im Blut über 24 Stunden nahezu konstant blieb, während die Summe aus dem freien Wirkstoff und dem inaktiven Metaboliten stark schwankt (vgl. Figuren 1 und 2). Dabei bewegen sich die gemessenen Blutkonzentrationen des freien Östriols in einem Bereich, der den physiologischen Konzentrationen östrogener Hormone im Zyklus der Frau (ca. 50 bis 350 pg/ml) entsprechen und die damit geeignet sind, Östrogenmangelzustände optimal zu beheben.

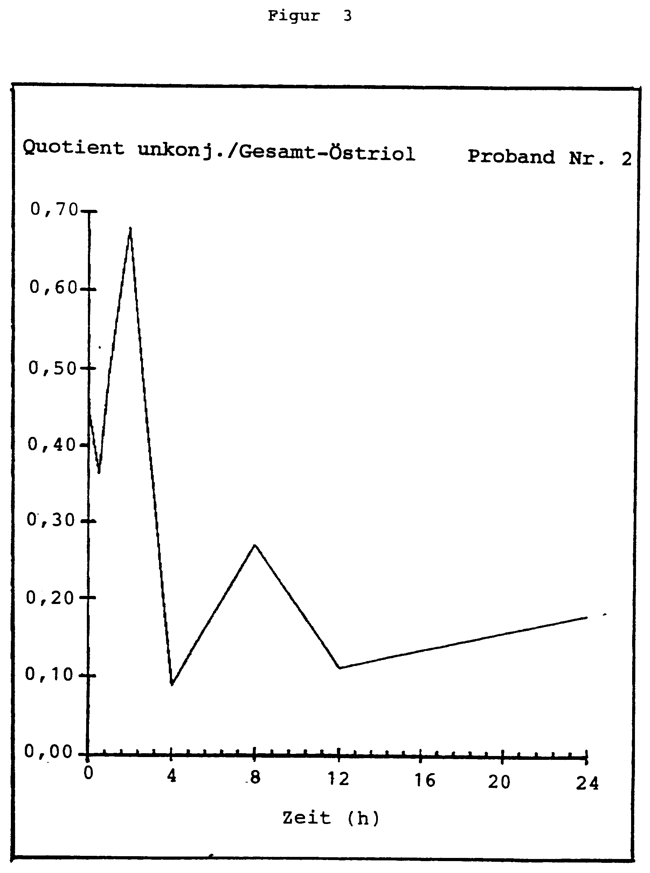

Die gleichbleibende Konzentration von freiem Östriol bei veränderlichem Gesamtöstriolspiegel läßt vermuten, daß bei kontinuierlicher Zufuhr ein Teil des metabolisierten Östriols wieder in die Ausgangssubstanz zurückverwandelt wird, wobei jedoch die Umwandlung in solcher Weise gesteuert zu sein scheint, daß letztlich ein konstanter Spiegel zustande kommt. Ein weiterer Nachweis dafür, daß der erfindungsgemäß beobachtete Effekt nicht linear konzentrationsabhängig ist sondern auf einem aktiven Regelmechanismus beruht ist der stark schwankende Quotient aus unkonjugiertem und Gesamt-Östriol (vgl. Fig.3 und 4). Für die erfindungsgemäße Verwendung liegt hierin gleichzeitig ein vorteilhafter Schutzmechanismus des Körpers gegen Überdosierung.

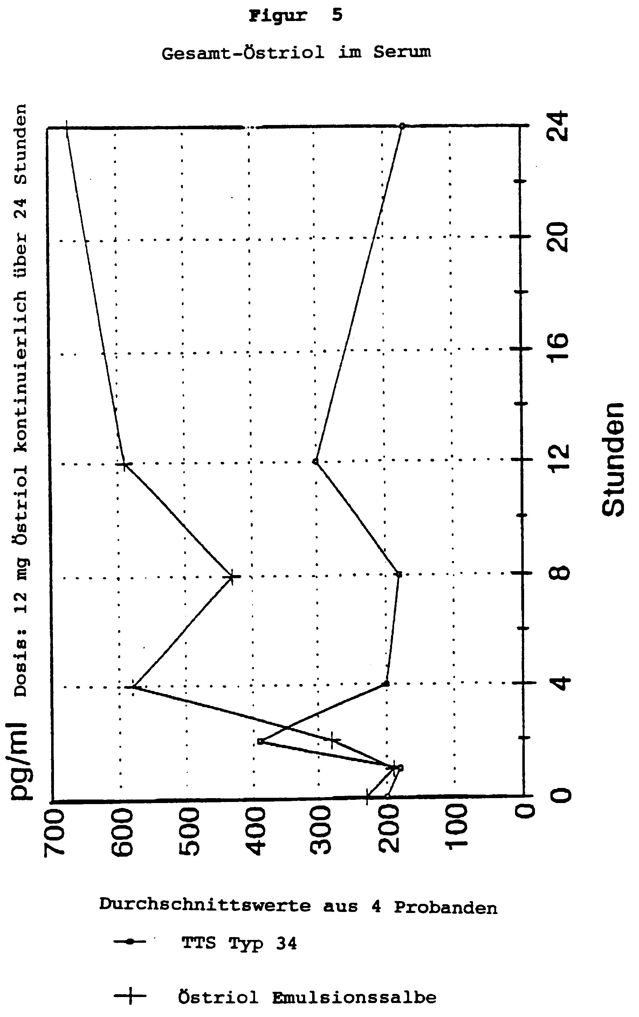

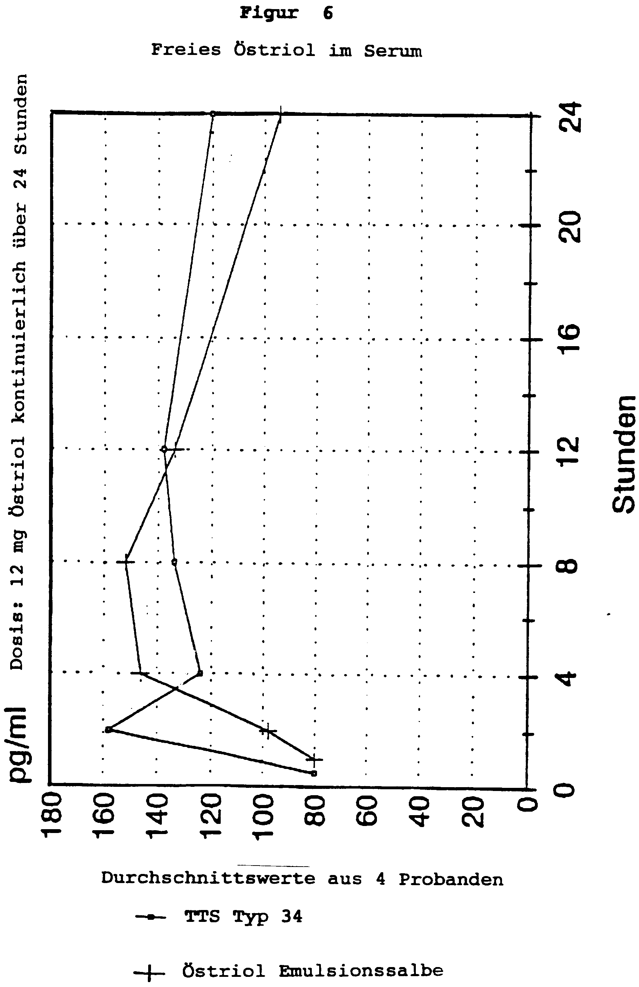

Der erfindungsgemäße Befund und die Vorteile der sich aus diesem herleitenden erfindungsgemäßen Lehre werden durch die Ergebnisse mit verschiedenen transdermalen Systemen (siehe unten) bestätigt. Während nämlich die Gesamtmengen aus freiem und metabolisiertem Wirkstoff systemabhängig um einen Faktor von 2 bis 3 schwankten, wurde gleichzeitig in völlig unerwarteter Weise ein praktisch identischer Verlauf der für die Wirksamkeit entscheidenden freien Östriolspiegel beobachtet (vgl. Fig. 5 und 6).

Im Gegensatz zu der bisher im Stand der Technik vertretenen Auffassung, daß es sich bei Östriol um ein **schwaches** Östrogen handele, hat es sich unter den erfindungsgemäßen Applikationsbedingungen gezeigt, daß Östriol ein **ausgesprochen starkes** Östrogen ist. Die erfindungsgemäßen Befunde sprechen für Östriol als das Östrogen der Wahl für die kontinuierliche HormonSubstitutions-Therapie und insbesondere für die Therapie klimakterischer Osteoporose. Bei kontinuierlicher Verabreichung wird nämlich einerseits Osteoporose wirksam therapiert bzw. präventiert, während andererseits die bei herkömmlichen Östrogenen beobachtete kanzerogene Wirkung entfällt und sogar eine antikanzerogene Wirkung erwartet werden kann.

Erfindungsgemäß sind im Prinzip alle transdermalen therapeutischen Systeme - **TTS** - geeignet, welche die kontinuierliche Wirkstofffreisetzung über mindestens 24 Stunden gewährleisten. Erfindungsgemäß besonders bevorzugt sind transdermale Wirkstoffpflaster oder transdermale Emulsionssalben. Bevorzugte Dosierungsformen sind solche, welche in 24 Stunden 5 bis 25, vorzugsweise 10 bis 15 und in besonders bevorzugter Weise 12 mg Östriol je Dosierungseinheit freisetzen.

Ein erfindungsgemäßes TTS in Form eines Wirkstoffpflasters ist vorzugsweise als einfaches Matrixsystem ausgebildet. Das bedeutet, daß die Wirkstoffe in einem Gerüst (Reservoir) enthalten sind, in der gegebenenfalls noch penetrationsverstärkende Mittel enthalten sein können. Die Matrix befindet sich auf einer Trägerfolie, vorzugsweise einer Aluminiumfolie, und sie ist mit einer abziehbaren Schutzschicht, oder einer undurchlässigen Deckschicht nach außen hin abgesschlossen. Sofern eine Deckschicht verwendet wird, kann diese gewünschtenfalls penetrationsverstärkende Mittel enthalten.

Ein einfaches Matrixsystem im Sinne der Erfindung kann beispielsweise wie folgt hergestellt werden:

Eine Lösung oder Suspension von 1 bis 60 Gew.% Wirkstoff wird unter Zusatz von 0 bis 45 Gew.% eines penetrationsverstärkenden Mittels und 30 bis 70 Gew.% eines Polyakrylatklebers zum Pflaster verarbeitet. Der Polyacrylatkleber kann in einem geeigneten flüchtigen Lösungsmittel gelöst oder in Wasser dispergiert vorliegen. Die Mischung aus Wirkstoff, Penetrationsförderer und Kleber wird auf eine plane undurchlässige Aluminiumfolie gestrichen, getrocknet und danach mit einer abziehbaren Schutzschicht, beispielsweise aus silikonisiertem Gewebe oder fluorpolymerbeschichtetem Papier, versehen.

Wird ein Polyacrylat verwendet, das nach dem Trocknen des Systems nicht oder nicht ausreichend auf der Haut haftet, so kann man das System vor dem Aufbringen der abziehbaren Schutzschicht noch zusätzlich mit einem Haftkleber abdecken oder umgeben. Üblicherweise wird als Material für diesen Haftkleber ebenfalls ein Polyacrylat verwendet. Diese Vorgehensweise hat den Vorteil, daß die Schichten bei der Herstellung oder in Folge einer nachträglichen Verbindung unter Druck (Kalandrieren) in engen Kontakt gebracht werden können. Auf diese Weise entsteht ein Schichtsystem (Laminat), in welchem und aus welchem der Wirkstoff günstig diffundieren kann.

In analoger Weise können jedoch als Materialien für die Matrixschicht und den Haftkleber auch Silicone, Polyurethane sowie natürliche oder synthetische Kautschuke verwendet werden. Als weitere Zusätze zur Matrix kommen Cellulosen und/oder Cellulosederivate, Polyvinylverbindungen, Silicone oder Silikate in Betracht.

Als transdermal wirksame Salben - **TwS** - werden erfindungsgemäß vorzugsweise W/O-Emulsionssysteme (Wasser/Öl-Emulsionssysteme) eingesetzt. Dies bedeutet, daß die Wirkstoffe in Form einer wäßrigen Lösung, die gegebenenfalls einen Penetrationsförderer enthalten kann, einer in der Pharmazie üblichen W/O-Grundlage zugesetzt werden. Die komplette Salbe befindet sich in Volumina, die jeweils einer einzelnen Dosierung entsprechen, in Näpfen, die auf einer geeigneten Folie, vorzugsweise einer Kunststoffolie, ausgeformt sind. Die Näpfe sind mit einer abziehbaren Schutzschicht verschlossen. Die fertige Packung ist einer "Blisterpakkung" im Prinzip ähnlich.

Als einfache W/O-Grundlage im Sinne der Erfindung kann beispielsweise die Wollwachsalkoholsalbe des DAB 10 eingesetzt werden. Wird ein geeigneter Emulgator verwendet, so können auch kompliziertere Emulsionen entstehen. In Betracht kommen auch O/W/O- oder W/O/W/O-Systeme, soweit sie die erfindungsgemäßen Voraussetzungen, nämlich eine kontinuierliche Wirkstoffabgabe über mindestens 24 Stunden, erfüllen. Gelegentlich kann es vorkommen, daß die Emulsion nicht oder nicht ausreichend auf der Haut haftet oder in diese eindringt. In diesem Falle können der äußeren Phase noch zusätzlich geeignete haftvermittelnde Zusätze oder penetrationsfördernde Mittel zugesetzt werden. Beispielsweise wird als penetrationsförderndes Mittel ein ein- oder mehrwertiger Alkohol verwendet. Bei der Herstellung der Salbe kann von einer Wirkstofflösung oder Suspension ausgegangen werden, die 1 bis 60 Gew.% Östriol enthältund diese kann mit bis zu etwa 12 Gew.% Stabilisator verdickt sein. Als Stabilisatoren können beispielsweise Alluminiumstearat, Bentonit, Kieselsäure oder ein Siliziumdioxid dienen.

Die Erfindung wird nachfolgend anhand von Beispielen erläutert.

### Beispiel 1

### Formulierung eines transdermal wirksamen (TTS-Typ 22) Pflasters

Das Pflaster wurde aus zwei Ansätzen der nachfolgend angegebenen Zusammensetzung hergestellt:

| Ansatz I | |
|---|---|
| Bestandteile | mg/Pflaster |
| Östriol | 12 |
| Ethanol | 70 |
| Wasser | 18 |

| Ansatz II | |
|---|---|
| Bestandteile | mg/Pflaster |
| Eudragit® L 100 | 14 |
| Eudragit® S 100 | 14 |
| Farbstoff | 2 |
| Glycerol | 5 |
| Wasser | 65 |

Ansatz I und Ansatz II werden gemischt. Die Mischung in geeigneter Weise z.B. im Gießverfahren oder durch Rakelauftrag, dünn ausgestrichen und zum Film getrocknet. Auf den getrockneten Film wird in dünner Schicht eine geeignete Haftschicht (z.B. aus Durotac®) aufgebracht, so daß ein Laminat entsteht. Danach wird das Laminat erneut getrocknet.

Es ist auch möglich, das Laminat im sogenannten "Transferverfahren" durch Kalandrierung des Filmes, der die Arzneistoffe enthält, mit einem geeigneten Haftfilm herzustellen. Die Haftschicht kann vor der Anwendung des Transdermalpflasters in bekannter Weise mit einer Schutzfolie bedeckt sein.

Das Transdermalpflaster haftet auf der menschlichen Haut, auch wenn diese leicht behaart ist. Die enthaltenden Wirkstoffe können durch den Haftfilm diffundieren, in die Haut eindringen und systemisch wirksam werden.

### Beispiel 2

### Formulierung eines transdermal wirksamen Pfasters (TTS-Typ 34)

Die Vorgehensweise gemäß Beispiel 1 wurde wiederholt, mit dem Unterschied, daß anstelle von Eudragit® L100 und S100 insgesamt 28 mg Softisan® TSD 34M15/3A verwendet wurden.

### Beispiel 3

### Herstellung einer transdermal wirksamen Emulsionssalbe

Eine undurchlässige Folie wurde durch Wärme und/oder Zug so verformt, daß eine 0,1 bis 3 ml fassende Ausbuchtung entstand. Diese wurde mit der Salbe gefüllt. Die Salbe enthielt eine wirkstoffhaltige Lösung bzw. Suspension in einer Konzentration von 12 mg Östriol je 0,5 g Salbe. Letztere wurde aus zwei verschiedenen Ansätzen wie nachfolgend angegeben hergestellt:

| Ansatz I | |
|---|---|
| Bestandteile | mg/0,5 g Salbe |
| Östriol | 12 |
| Ethanol | 7 |
| Wasser | 81 |

| Ansatz II | |
|---|---|
| Bestandteile | mg/0,5 g Salbe |
| Wasser | 30 |
| Glycerol mittelkettige | 15 |
| Triglyceride | 55 |
| Wollwachsalkoholsalbe | 400 |

Ansatz II wurde homogenisiert und im Wasserbad auf 60°C erwärmt. Anschließend wurde der auf die gleiche Temperatur erwärmte Ansatz I anteilweise in die homogenisierte Mischung eingearbeitet.

Jeweils 0,5 g Salbe werden in eine, wie oben beschrieben ausgeformte Vertiefung einer Folie (Blister) eingefüllt und verschlossen.

### Beispiel 4

### Untersuchung der osteoporotischen Wirkung von Östriol bei ovarektomierten Ratten

1. Fragestellung
   11 Wochen alte weibliche Wistarratten verlieren einen erheblichen Anteil ihrer Knochensubstanz in zwei Wochen nach Ovariektomie. Es sollte untersucht werden, ob dieser Effekt durch Östriol verhinderbar ist. Hierbei sollte dieses Hormon so appliziert werden, daß über den gesamten Versuchszeitraum gleichmäßige Hormonspiegel eingestellt werden. Diesem Vorgehen lag die Hypothese zugrunde, daß das bisher beobachtete Ausbleiben einer Wirkung am Knochen unter Östriolbehandlung unter Umständen auf Unterschreitung eines kritischen Hormonspiegels im Blut zwischen zwei Applikationszeitpunkten bei Behandlungsformen, wie z.B. täglicher oraler Applikation beruhte. Als weitere Möglichkeit wäre die Annahme zu prüfen, daß Östriol als partieller Östrogenantagonist am Knochen unzureichende Aktivität besitzt.
2. Material und Methoden
   Weibliche Wistarratten der Versuchstierzucht "Biomodelle GmbH", O-1291 Schönwalde (Stamm: Wistar) wurden im Alter von neun bis zehn Wochen angeliefert und unter konventionellen Bedingungen bei einer Raumtemperatur von 22°C +/- 24°C und unter einem Lichtregime (14 h hell und 10 h dunkel) in PVC-Schalen mit Drahtdeckelaufsatz gehalten.
   Die Tiere erhielten Standardfutter der Rezeptur R 13 sowie Trinkwasser ad libitum.
   Im Alter von 11 Wochen (Körpermasse ca. 220 g) kamen die Tiere in den Versuch.
3. Applikation der Testsubstanz
   Subkutane Applikation osmotischer Pumpen. Typ: Alzet 2002, Volumen des Reservoirs 200 µl. Durchfluß 0,5 µl/Stunde. Lösung der Testsubstanz in Propylenglykol.
   Die gesamte Versuchsdauer betrug 3 Wochen, 1 Woche vor der Kastration und 2 Wochen (15 Tage) nach der Kastration.
   In der Woche vor der Kastration wurden charakteristische Parameter vor dem östrogenbedingten Knochenabbau durch Ovarektomie als "Ausgangswerte" ermittelt (Hydroxyprolingehalt im 24-h-Harn, Hydroxyprolingehalt und Östriolkonzentration im Serum).
   Zur Ermittlung des Hydroxyprolingehaltes im Harn wurde Sammelurin über 24 Stunden gewonnen. Dazu wurden die Tiere wöchentlich einmal einzeln in Diuresekäfige gesetzt, die eine hinreichend saubere, vom Kot getrennte Harnsammlung gestatten. Im Sammelharn von jedem Einzeltier erfolgte dann die Ermittlung des Hydroxyprolingehaltes.
   Im Serum, das durch Blutentnahme aus dem retrobulbären Venenplexus am nullten, siebenten und vierzehnten Tag nach der Kastration gewonnen wurde, wurden Hydroxyprolin- und Östriolspiegel ermittelt.
4. Untersuchung der Effekte von Östriol auf den Knochen
   Bei Versuchsende wurden die Tiere getötet und die Femurknochen für histologische Untersuchungen gemäß Standardmethode fixiert, mit einem Hartschnittmikrotom geschnitten und im Hinblick auf morphologische Merkmale qualitativ und quantitativ untersucht.
5. Versuchsergebnisse
   Die Versuchsergebnisse sind in den Tabellen 1 bis 4 dargestellt.
6. Bewertung
   Der Ausgang der Versuche zeigt, daß - anders als in der Literatur für Östriol beschrieben - diese Substanz ein osteoprotektives Potential besitzt.

Voraussetzung für diese Wirkung ist nach den vorliegenden Versuchen die Erzeugung gleichmäßiger Wirkstoffspiegel. Die erzielten Ergebnisse führten zu der erfindungsgemäßen vorgeschlagenen Östrioltherapie.

**Tabelle 1**

| **Versuchsgruppen** | | | | |
|---|---|---|---|---|
| Versuchsgruppenbezeichnung | N | Konditionierung | Behandlung | Dosis/24 Std. |
| SHO + V (intakte Kontrollen) | 8 | Schein OP | Propylenglykol | |
| OVX + V (Kontrolle) | 8 | kastr. | " | |
| OVX + D1 | 8 | kastr. | Östriol | 0,1 µg |
| OVX + D2 | 8 | kastr. | in | 1,0 µg |
| OVX + D3 | 8 | kastr. | Propylenglykol | 10,0 µg |
| OVX + D4 | 9 | kastr. | | 50,0 µg |

### Ergebnisse

1. Hydroxyprolinausscheidung im Harn, Hydroxyprolinspiegel im Serum
   Hydroxyprolin wird beim Abbau von Kollagen, das auch einen wesentlichen Anteil der Knochensubstanz ausmacht, freigesetzt.
   Nach der Ovarektomie wurden im Blut steigende Hydroxyprolinwerte erhoben (Tabelle 2), dieser Anstieg wurde durch die Behandlung mit Östriol dosisabhängig unterdrückt.
   Ebenso steigt nach der Kastration die Ausscheidung von Hydroxyprolin im Harn an. Auch dieser Effekt ist durch die Behandlung mit Östriol aufhebbar (Tabelle 3).

**Tabelle 2**

| **Hydroxyprolingehalt [µmol/1 l] im Serum scheinoperierter (SHO) und kastrierter (OVX) Wistarratten nach Estriolapplikation (durch Bolusimplantation)** | | | | |
|---|---|---|---|---|
| Dosierung | | Tage nach Kastration | | |
| | | 0 | 7 | 15 |
| SHO + V (n = 8) | X | 30,59 | 28,05 | 25,04 |
| | s | 4,46 | 2,82 | 5,50 |
| relative Änderung [%] | X | | -6,47 | -16,69 |
| OVX + V (n = 8) | X | 29,92 | 40,35 | 39,41 |
| | s | 4,32 | 3,71 | 3,72 |
| relative Änderung [%] | X | | 36,71 | 33,77 |
| OVX + D1 (n = 8) | X | 30,55 | 35,31 | 33,36 |
| | s | 4,78 | 4,11 | 6,91 |
| relative Änderung [%] | X | | 18,44 | 11,66 |
| OVX + D2 (n = 8) | X | 30,74 | 33,20 | 30,51 |
| | s | 3,03 | 3,63 | 5,10 |
| relative Änderung [%] | X | | 8,82 | 0,39 |
| OVX + D3 (n = 8) | X | 28,16 | 26,25 | 20,90 |
| | s | 4,85 | 3,52 | 1,58 |
| relative Änderung [%] | X | | -3,56 | -23,81 |
| OVX + D4 (n = 9) | X | 28,26 | 25,45 | 17,33 |
| | s | 3,17 | 3,85 | 3,32 |
| relative Änderung [%] | X | | -8,87 | -38,62 |
| n* = Tierzahl X = Mittelwert s = Standardabweichung | | | | |

**Tabelle 3**

| **Hydroxyprolingehalt [µmol/d] im Harn scheinoperierter (SHO) und kastrierter (OVX) Wistarratten nach Estriolapplikation (durch Bolusimplantation)** | | | | | | | |
|---|---|---|---|---|---|---|---|
| Dosierung | | Tage nach Kastration | | | | | |
| | | 0 | 7 | 12 | 13 | 14 | 15 |
| SHO + V (n = 8) | X | 1,62 | 1,67 | 1,91 | 1,40 | 1,75 | 1,10 |
| | s | 0,38 | 0,83 | 0,63 | 0,93 | 0,65 | 0,43 |
| relative Änderung [%] | X | | 4,34 | 22,02 | -17,13 | 6,83 | -29,13 |
| OVX + V (n = 8) | X | 1,82 | 1,92 | 2,80 | 2,35 | 2,81 | 1,85 |
| | s | 0,42 | 0,48 | 0,85 | 1,02 | 1,03 | 1,51 |
| relative Änderung [%] | X | | 7,58 | (n = 7) 57,30 | 26,73 | 59,37 | 4,69 |
| OVX + D1 (n = 8) | X | 1,46 | 2,15 | 2,41 | 2,51 | 2,11 | 2,00 |
| | s | 0,46 | 0,75 | 0,93 | 0,72 | 0,87 | 0,85 |
| relative Änderung [%] | X | | 56,95 | 84,62 | 79,03 | 59,47 | 40,95 |
| OVX + D2 (n = 8) | X | 1,63 | 1,84 | 2,97 | 2,40 | 2,31 | 1,83 |
| | s | 0,36 | 0,57 | 1,36 | 1,05 | 1,23 | 0,85 |
| relative Änderung [%] | X | | 15,48 | 74,86 | 42,71 | 37,83 | 10,69 |
| OVX + D3 (n = 8) | X | 1,67 | 0,88 | 0,98 | 1,04 | 0,84 | 1,16 |
| | s | 0,20 | 0,47 | 0,27 | 0,22 | 0,34 | 0,62 |
| relative Änderung [%] | X | | 47,88 | -40,47 | -36,63 | -48,91 | -28,45 |
| OVX + D4 (n = 8) | X | 1,53 | 0,99 | 1,09 | 1,06 | 0,98 | 0,93 |
| | s | 0,40 | 0,36 | 0,57 | 0,37 | 0,21 | 0,30 |
| relative Änderung [%] | X | (r = 9) | (n = 9) -34,52 | -33,26 | -35,04 | -38,21 | -40,43 |

**Tabelle 4**

| **Effekte einer Östriolbehandlung auf den trabekulären Anteil des Knochenvolumens nach Ovarektomie** | |
|---|---|
| Behandlung | % trabekulärer Anteil |
| Schein OP, Vehikelbehandlung (= intakte Kontrolle) | 25,44 |
| Ovarektomie (OVX), Vehikelbehandlung | 14,40 |
| OVX + 0,1 Mikrog E₃¹⁾/Tag | 16,89 |
| OVX + 1,0 Mikrog E₃¹⁾/Tag | 17,46 |
| OVX + 10,0 Mikrog E₃¹⁾/Tag | 24,79 |
| OVX + 50,0 Mikrog E₃¹⁾/Tag | 26,64 |

| | |
|---|---|
| ¹⁾ E₃ = Östriol | |

### 2. Effekte auf die Knochensubstanz:

Als Parameter der osteoprotektiven Wirkung von Östriol wurde der Effekt auf den Anteil trabekulärer Strukturen in der Fläche von Knochenschnitten bestimmt. Aus Tabelle 4 geht hervor, daß ein erheblicher Anteil der Knochenmasse nach einer Kastration abgebaut wird. Durch Östriol wurde dieser Effekt dosisabhängig aufgehoben.

### Beispiel 5

### Pilotstudie zum Blutspiegelverlauf von Östriol nach transdermaler Applikation an gesunden männlichen Probanden

Ziel der Studie war es, die Östriol-Serumkonzentration bei kontinuierlicher Verabreichung aus verschiedenen transdermalen Systemen über Stunden zu untersuchen. Der Studientyp entsprach einer klinischen Prüfung Phase I (Pilotstudie), die von der Ethik-Kommission der Friedrich Schiller Universität Jena begutachtet worden war und ein positives Votum für die Durchführung erhalten hatte.

Die klinische Prüfung wurde an gesunden Männern durchgeführt, da Gegenstand der Untersuchung die pharmazeutischen Eigenschaften der Formulierung waren und nicht der Metabolismus der Wirksubstanz im Organismus. Alle Faktoren, die hinsichtlich Absorption, Distribution, Metabolismus und Elimination die Vergleichbarkeit stören könnten, mußten also ausgeschlossen werden. Deswegen waren Personen mit eigenem und möglicherweise schwankendem Basalspiegel des zu untersuchenden Hormons, d.h., weibliche Probandinnen, für diese Studien nicht geeignet.

### Prüfpräparate:

Es wurden drei verschiedene transdermale Formulierungen überprüft, die jeweils 12 mg Estriol enthielten:
1. TTS-Typ 22:
   Pflaster mit einem Durchmesser von 32 mm, Deckschicht aus Aluminium, Östriol-Reservoir und Haftschicht aus Polyacrylat (Reservoir: Scopacryl®, Buna GmbH; Haftschicht: Durotac®, Delft National Corp.). Das Östriol war in dem Reservoir und teilweise in der Haftschicht molekular dispers verteilt.
2. TTS-Typ 34:
   Plaster mit einem Durchmesser von 56 mm, Deckschicht aus fleischfarbenem Gewebe, Östriolreservoir als Scheibe mit einem Durchmesser von 36 mm im Zentrum, Östriolreservoir besteht aus einem schwammartigen Material (Softisan® TSD 34M15/3A), in welchem Östriol fein verteilt vorliegt, Haftschicht aus üblichem Pflastkleber.
3. Östriol-TwS:
   Creme mit einer Wirkstoffkonzentration von 12 mg Östriol/0,5g.

### Art und Umfang der Studie:

Es wurden drei Gruppen aus jeweils 4 gesunden Männern im Alter zwischen 18 und 55 Jahren gebildet. Jeder Gruppe wurde eines der Prüfpräparate zugeteilt, wobei die Pflaster im Bereich des Gesäßmuskels geklebt bzw. das Gel im Stirnbereich einmassiert wurde. Die Prüfpräparate wurden zeitlich parallel verabreicht und die Gruppen entsprechend einer Randominiserungsliste zugeordnet. Die Vergabe der Randomnummer erfolgte sequentiell entsprechend der Reihenfolge der Aufnahme der Probanden in die Studie. Es wurden Blutproben zu den Zeitpunkten 0, 0,5, 1, 2, 4, 8, 12 und 24 entnommen.

### Untersuchungsablauf:

Die Verabreichung der Prüfpräparate erfolgte am Tag der Prüfung morgens um 8 Uhr. Zur Beurteilung des Östriolplasmaspiegels wurde zu den angegebenen Zeitpunkten jeweils 10 ml Blut abgenommen, mittels Zentrifugation das Serum abgetrennt und bis zur Bestimmung der Hormonkonzentration bei -20°C gelagert.

### Hormonbestimmung:

Die Bestimmung von Gesamtöstriol und freiem Östriol erfolgte mittels RIA-Technik (Amersham-RIA) im Hormonlabor der Universitätsfrauenklinik Tübingen. Zur Bestimmung des Gesamt-Östriolspiegels wurden die Seren initial mit Glucoronidase/Sulfatase-Lösung inkubiert und das freigesetzte dikonjugierte Östriol mit Ether extrahiert. Die RIA-Bestimmung wurde dann mit den im Puffer aufgenommenen Extrakten durchgeführt. Freies Östriol wurde aus nicht vorbehandelten Proben direkt extrahiert und auf gleiche Weise bestimmmt.

### Auswertung:

Bestimmt wurde die Konzentration von freiem und Gesamtöstriol im Serum der Probanden. Das Gesamtöstriol schließt freies und metabolisiertes Östriol ein. Aus dem zeitlichen Verlauf wurden für jeden Probanden folgende Parameter bestimmt:
- AUC:: Fläche unter der Konzentrationszeitkurve, die mit der Trapezregel errechnet wird (Zeitpunkt 0 bis 24 Stunden)
- Cₘₐₓ:: Konzentrationsmaximum
- Tₘₐₓ:: Zeit bis zum Erreichen des Maximums

### Ergebnisse:

Die Studie führte zu dem unerwarteten Ergebnis, daß die Kurve der gesamten im Blut befindlichen Menge an Wirkstoff und metabolisiertem Wirkstoff (Gesamtöstriol im Serum) nach transdermaler Applikation aller drei Systeme zwischen den verschiedenen Meßzeitpunkten einen unregelmäßigen Verlauf zeigt (vgl. Figuren 1 und 2, entsprechend Probanden 2 und 7). Völlig überraschend ist es unter diesen Bedingungen, daß nach initialem Ansteigen die Konzentration des therapeutisch wirksamen, nicht-metabolisierten Hormons (unkonjugiertes Östriol) über 24 Stunden nahezu konstant bleibt (Figuren 1 und 2 entsprechend Probanden 2 und 7), während der Quotient aus freiem und Gesamt-Östriol stark schwankt (Figuren 3 und 4 entsprechend Probanden 2 und 7).

Dabei bewegen sich interessanterweise die gemessenen Blutkonzentrationen des freien Östriols in dem Bereich, der physiologischen Konzentrationen östrogener Hormone im Zyklus der Frau (ca. 50 bis 350 pg/ml) entspricht. Es ist deshalb davon auszugehen, daß alle untersuchten transdermalen Systeme geeignet sind, Östrogenmangelzustände optimal zu beheben.

Dies gilt umso mehr, als die verschiedenen Formulierungen zu verschiedenen Gesamtserum-Östriolkonzentrationen führten (vgl. Figur 5), wobei die Konzentrationen des transdermalen Retard-Emulgels um einen Faktor 2 bis 3 oberhalb des Systems TTS Typ 34 lagen, überraschenderweise jedoch ein praktisch identischer Verlauf der allein relevanten Östriolspiegel (nicht-konjugiertes Serum Östriol) beobachtet wurde, vgl. Figur 6.

Das Ergebnis der oben beschriebenen Pilotstudie legt die Vermutung nahe, daß bei kontinuierlicher Verabreichung von Östriol endogene Mechanismen "regulierend" auf den gemessenen Östriolspiegel im Blut einwirken, was zu offensichtlichen Vorteilen bei der Wahl von Östriol für die Hormon-Substitutions-Therapie und insbesondere zur Therapie der klimakterischen Osteoporose führt, da beispielsweise eine Überdosierung weitgehend ausgeschlossen zu sein scheint.

## Patentansprüche

1. Verwendung von Östriol zur Herstellung von mindestens 24 Stunden lang kontinuierlich Wirkstoff freisetzenden transdermalen therapeutischen Systemen zur Behandlung von klimakterischer Osteoporose, wobei die Systeme Östriol als alleinigen Wirkstoff enthalten.

2. Verwendung nach Anspruch 1, **dadurch gekennzeichnet**, daß das transdermale therapeutische System ein transdermales Pflaster ist.

3. Verwendung nach Anspruch 1, **dadurch gekennzeichnet**, daß transdermale therapeutische System eine transdermale Emulsionssalbe ist.

4. Verwendung nach den Ansprüchen 1 bis 3, dadurch gekennzeichnet, daß das transdermale therapeutische System je Dosiseinheit 5 bis 25 mg Östriol enthält.

## Claims

1. Use of estriol for the production of transdermal therapeutic systems which release active ingredient continuously for at least 24 hours for the treatment of climacteric osteoporosis, the systems containing estriol as sole active ingredient.

2. Use according to claim 1, characterized in that the transdermal therapeutic system is a transdermal plaster.

3. Use according to claim 1, characterized in that the transdermal therapeutic system is a transdermal emulsion ointment.

4. Use according to claims 1 to 3, characterized in that the transdermal therapeutic system contains 5 to 25 mg estriol per dose unit.

## Revendications

1. Utilisation d'oestriol pour la production de systèmes thérapeutiques transdermiques libérant un agent actif continuellement pendant au moins 24 heures pour le traitement de l'ostéoporose due à la ménopause, les systèmes contenant de l'oestriol comme seul agent actif.

2. Utilisation selon la revendication 1, **caractérisée en ce que** le système thérapeutique transdermique est un emplâtre transdermique.

3. Utilisation selon la revendication 1, **caractérisée en ce que** le système thérapeutique transdermique est une pommade en émulsion transdermique.

4. Utilisation selon les revendications 1 à 3, **caractérisée en ce que** le système thérapeutique transdermique contient, par dose unitaire, 5 à 25 mg d'oestriol.
